# EUROPÄISCHE PATENTANMELDUNG

(11) **EP 4 074 783 A1**
(43) Veröffentlichungstag der Anmeldung: **19.10.2022**
(21) Anmeldenummer: 21167981.6
(22) Anmeldetag: 13.04.2021
(51) Int. Cl.: C09D 5/14, A61L 2/08, A61L 2/10, C09D 5/22, C09K 11/77

(54) **WASSERBASIERTE HÄRTBARE ZUSAMMENSETZUNG ZUR HERSTELLUNG VON BESCHICHTUNGEN MIT LEUCHTSTOFFEN**

(71) Anmelder: Evonik Operations GmbH, 45128 Essen (DE)
(72) Erfinder: SCHULTE, Simone, 45133 Essen (DE); HALLACK, Markus, 46514 Schermbeck (DE); HUTH, Michael, 63477 Maintal (DE); TSCHERNJAEW, Juri, 63743 Aschaffenburg (DE); FISCHER, Stefan, 59494 Soest (DE); JÜSTEL, Thomas, 58455 Witten (DE)
(74) Vertreter: Evonik Patent Association

(57) **Zusammenfassung**

Die Erfindung betrifft eine wasserbasierte härtbare Zusammensetzung zur Herstellung von Beschichtungen mit anti-mikrobieller Eigenschaft enthaltend
- mindestens ein filmbildendes Polymer,
- optional mindestens ein Additiv,
- optional mindestens einen Härter,
- mindestens einen up-conversion Leuchtstoff der allgemeinen Formel (I)

A_{1-x-y-z}B*_{y}B₂SiO₄:Ln¹ₓ,Ln²_{z}, I

mit
x = 0,0001 - 0,0500;
z = 0,0000 oder z = 0,0001 bis 0,3000 mit der Maßgabe, dass gilt: y = x + z;
A ausgewählt aus der Gruppe bestehend aus Mg, Ca, Sr und Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
Ln¹ ausgewählt aus der Gruppe bestehend aus Praseodym (Pr), Erbium (Er) und Neodym (Nd);
Ln² ausgewählt aus Gadolinium (Gd),
wobei der Leuchtstoff durch eine Nachbehandlung mindestens ein Material, das eine Bandlücke von größer als 6,0 Elektronenvolt (eV) hat und hydrolysestabil ist, aufweist.

## Beschreibung

Die Erfindung betrifft eine wasserbasierte härtbare Zusammensetzung zur Herstellung von Beschichtungen mit anti-mikrobieller Eigenschaft, deren Verwendung und daraus hergestellte Beschichtungen und Gegenstände, die damit beschichtet sind.

Tagtäglich sind Menschen einer großen Vielfalt von Mikroorganismen wie Bakterien, Pilzen und Viren ausgesetzt. Viele dieser Mikroorganismen sind nützlich bzw. sogar notwendig. Dennoch gibt es neben den harmloseren Vertretern auch krankheitsverursachende oder sogar tödliche Bakterien, Pilze und Viren.

Durch den täglichen Umgang mit anderen Menschen und den Kontakt mit Gegenständen, die andere benutzt haben, können Mikroorganismen übertragen werden. Die antimikrobielle Ausstattung von Oberflächen erfolgt insbesondere in hygienesensiblen Bereichen. Anwendungsbereiche sind vor allem Oberflächen von medizintechnischen Geräten und Bedarfsgegenstände in Krankenhäusern sowie in Einrichtungen des ambulanten Gesundheits- und Sozialwesens. Hinzu kommen Oberflächen im öffentlichen Raum, im Lebensmittelsektor und in der Tierhaltung. Die Verbreitung von pathogenen Mikroorganismen stellt heute ein großes Problem im Pflegebereich und in der Medizin dar, und auch überall dort, wo viele Menschen auf engem Raum verkehren. Ein besonders Risiko stellt gegenwärtig auch das vermehrte Aufkommen sogenannter multiresistenter Keime dar, die unempfindlich gegen den meisten Antibiotika geworden sind.

Um das Risiko der Verbreitung von pathogenen Erregern über Berührungsoberflächen zu verringern, werden, neben Maßnahmen zur Standardhygiene, antimikrobielle Technologien und Werkstoffe genutzt. Chemische Substanzen oder die Verwendung physikalischer Verfahren können den Vermehrungsprozess von Mikroorganismen kritisch beeinflussen. Zu den physikalischen Methoden zählen z. B. Hitze, Kälte, Strahlung oder Ultraschall, etc. Bei den chemischen Methoden sind Halogene, Metallionen, organische Verbindungen und Farbstoffe, bestimmte Gase wie Ozon usw. bekannt.

Obwohl in den meisten Fällen chemische und physikalische Methoden außerordentlich effektiv bei der Zerstörung von Mikroorganismen sind, so haben sie doch nur einen kurzzeitigen Effekt, chemische Methoden fördern die Entstehung von Resistenzen und sind unter Umständen für manche Anwendungen nicht geeignet, da sie zur Zerstörung der zu schützenden Oberflächen führen. Den größten Nachteil stellt allerdings, gerade bei chemischen, organischen Substanzen, die Gefährlichkeit bzw. Giftigkeit für den Menschen dar. Bestimmte Substanzen, wie z. B. Formaldehyd, welches viele Jahre als Desinfektionsmittel Anwendung fand, stehen inzwischen im Verdacht, Krebs zu verursachen oder extrem umweltschädlich zu sein.

Oberflächen mit antimikrobieller Wirkung könnten einen entscheidenden Beitrag zur Lösung dieser Probleme leisten. Die heute gängigen Verfahren zur Erzeugung solcher antimikrobiellen Eigenschaften verwenden überwiegend in das Material eingearbeitete Wirkstoffe wie z.B. Silberpartikel, Kupferpartikel, deren Metalloxide oder quaternäre Ammoniumverbindungen. Dabei werden die antimikrobiell wirkenden Metalle, Metalloxide oder Metalloxidgemische häufig zu Nanoteilchen verarbeitet und dann Farben, Lacken oder Polymerwerkstoffen zugemischt. Der breite Einsatz von Metallpartikeln ist in Frage zu stellen, da die langfristige Wirkung dieses Schwermetalls auf Menschen und Umwelt kaum abzuschätzen ist.

Beispielsweise offenbart die WO 2019/197076 Partikel, die mit einer Schicht ausgestattet sind, die sowohl Antimon-Zinnoxid als auch Manganoxid enthält. Dem Fachmann ist bekannt, dass die antimikrobiellen Oberflächen aufgrund des elektrochemischen Verhaltens von Metallen erzeugt werden, die bei Anwesenheit von Feuchtigkeit mikrogalvanische Zellen und durch die mikroelektrischen Felder keimabtötende Wirkung entfalten.

Es ist ebenso bekannt, UV-Strahlung in der Medizin oder in der Hygiene einzusetzen, um beispielsweise Wasser, Prozessgase, Luft oder Oberflächen zu desinfizieren. So wird in der Trinkwasseraufbereitung seit langem UV-Strahlung zur Reduzierung der Anzahl von fakultativ krankheitserregenden Mikroorganismen im Wasser eingesetzt. Dabei wird vorzugsweise UV-C-Strahlung im Wellenlängenbereich zwischen 200 nm und 280 nm eingesetzt. Der Einsatz elektromagnetischer Strahlung mit unterschiedlichen Wellenlängen sollte die unterschiedliche Absorption der verschiedenen Proteine, den in Mikroorganismen, Geweben oder Zellen enthaltenden Amino-/Nukleinsäuren (z.B. DNA oder RNA) sowie Peptidbindungen zwischen den einzelnen Säuren berücksichtigen. So absorbiert DNA/RNA elektromagnetische Strahlung innerhalb des Wellenlängenbereichs zwischen 200 und 300 nm gut und zwischen 250 und 280 nm besonders gut, so dass dieser Strahlungsbereich zur Inaktivierung bzw. Mutation von DNA/RNA besonders geeignet ist. Es können also krankheitserregende Mikroorganismen (Viren, Bakterien, Hefen, Schimmelpilze, Sporen u.a.) mit einer solchen Bestrahlung inaktiviert werden. Je nach Dauer und Intensität der Bestrahlung kann es zu Mutationen kommen oder sogar die Struktur von DNA bzw. RNA zerstört werden. Somit werden stoffwechselaktive Zellen inaktiviert und/oder deren Vermehrungsvermögen kann beseitigt werden. Vorteilhaft bei der Bestrahlung mit UV-Strahlung ist, dass die Mikroorganismen keine Resistenz dagegen entwickeln können. Diese physikalischen Methoden erfordern allerdings spezielle Apparaturen und müssen in der Regel von geschultem Personal regelmäßig wiederholt werden, was einen breiten Einsatz dieser Methoden erschwert.

Des Weiteren ist es neben einer direkten Bestrahlung mit elektromagnetischer Strahlung aus dem Wellenlängenbereich der UV-Strahlung auch bekannt, den Effekt der so genannten up-conversion auszunutzen. Dabei werden Leuchtstoffpartikel eingesetzt, mit denen elektromagnetische Strahlung mit Wellenlängen oberhalb des UV-Strahlung, insbesondere sichtbares Licht oder Infrarotstrahlung, in elektromagnetische Strahlung mit geringerer Wellenlänge gewandelt werden kann, so dass die Emission von in gewünschter Form wirkender Strahlung von den einzelnen Leuchtstoffpartikeln erreicht werden kann.

DE 10 2015 102 427 betrifft einen im Wellenlängenbereich der UV-Strahlung emittierenden Körper. Bei dem Körper sind in einem oberflächennahen Bereich innerhalb des Werkstoffs, aus dem der Körper gebildet ist oder einer Beschichtung auf dem Körper, Leuchtstoffpartikel eingebettet. Hierbei wird nur allgemein angegeben, dass die Leuchtstoffpartikel einer auf dem Werkstoff auszubildenden Beschichtung direkt bei der Verarbeitung zugegeben werden, dabei sollte der jeweilige Werkstoff eine geeignete Konsistenz bzw. Viskosität aufweisen. Hinsichtlich geeigneter Polymere und Additive schweigt DE 10 2015 102 427.

US 2009/0130169 A1 bzw. WO 2009/064845 A2 beschreibt Leuchtstoffe, die in Polyvinylchloride, Acrylbutadiene, Polyolefine, Polycarbonate, Polystyrole oder Nylon eingebracht werden können, welche durch die up-conversion Eigenschaft der Leuchtstoffe pathogene Mikroorganismen abtöten. Es handelt sich hierbei um Leuchtstoffe, die bei einer Temperatur von 1800 - 2900 °C hergestellt werden. Die US 2009/0130169 A1 bzw. WO 2009/064845 A2 offenbart zwar eine Zusammensetzung enthaltend besagter Leuchtstoffe mit behaupteter antimikrobieller Wirkung, zeigt jedoch weder einen Nachweis der up-conversion Eigenschaft noch mikrobiologischer Untersuchungen. Das darin offenbarte Verfahren führt zu keinem Leuchtstoff, der eine up-conversion Eigenschaft aufweist, sondern zu einem amorphen und glasartigen Produkt.
Darüber hinaus schweigt die US 2009/0130169 A1 bzw. WO 2009/064845 A2 über die Verträglichkeit der Komponente in der Beschichtungszusammensetzung oder die Eigenschaften der Beschichtungsoberflächen, wie etwa die Lackoberflächen. Das Erscheinungsbild von Beschichtungsoberflächen spielen jedoch beim Anwender eine besondere Rolle.

Die Anforderungen an Lacke und Farben sind vielfältig. Grundsätzlich haben Lack- bzw. Farbbeschichtungen zwei Aufgaben bzw. Funktionen, die schützende und die dekorative Funktion. Sollten im Weiteren nur der Begriff "Lackbeschichtung" aufgeführt sein, so sind beide Arten der Beschichtung gemeint. Sie verschönern, schützen und bewahren Materialien wie Holz, Metall oder Kunststoff. Demnach werden auf der einen Seite brillante und glatte Lackschichten gefordert, auf der anderen Seite eine geschlossene Lackschicht zur Sicherstellung der chemischen und mechanischen Beständigkeit, eine gewisse Gleitfähigkeit der Lacke oder eine besondere Haptik.

Im Gegensatz zu der WO 2009/064845 A2 konnten die noch nicht offengelegten Patentanmeldungen EP 19202910.6 und PCT/EP2020/077798 Leuchtstoffe und deren Herstellung offenbaren, die eine up-conversion aufweisen. Solche Leuchtstoffe können bei Bestrahlung mit elektromagnetischer Strahlung mit geringerer Energie und höherer Wellenlänge im Bereich von 2000 bis 400 nm, insbesondere im Bereich von 800 nm bis 400 nm, elektromagnetische Strahlung mit höherer Energie bzw. kürzerer Wellenlänge im Bereich von 400 bis 100 nm, bevorzugt im Bereich von 300 bis 200 nm emittieren, so dass sie geeignet sind, als anti-mikrobielle Leuchtstoffe in Lackbeschichtungen eingesetzt zu werden.

So beschreibt die noch nicht offengelegte Europäische Patentanmeldungen EP 21157055.1 eine Zusammensetzung enthalten mindestens ein filmbildendes Polymer, mindestens einen up-conversion Leuchtstoff gemäß der Lehre der EP 19202910.6 und PCT/EP2020/077798, optional mindestens ein Additiv und optional mindestens einen Härter. Es konnte gezeigt werden, dass Lackbeschichtungen enthaltend dieser Leuchtstoffe antimikrobiell wirken und ohne dass die übrigen Eigenschaften, insbesondere die Lagerstabilität, signifikant beeinträchtigt werden.

Es wurde jedoch auch festgestellt, dass die Leuchtstoffe, hergestellt nach einem Verfahren gemäß der EP 19202910.6 und PCT/EP2020/077798, sich nicht besonders gut für wasserbasierte Beschichtungssysteme eignen.

Aus dem Stand der Technik sind lösungsmittelbasierte, wasserbasierte und lösungsmittelfreie Beschichtungsstoffe bekannt.

Lösemittel werden eingesetzt, um eine verarbeitungsfähige Konsistenz von Beschichtungsstoffen einzustellen. In der Regel verwendet man niedermolekulare, organische Flüssigkeiten, in denen die eingesetzten Filmbildner vollständig löslich sind.

Beschichtungen, Beschichtungsstoffe, Lacke, Farben und Beschichtungssysteme werden hier als Synonyme verwendet.

Lösungsmittelhaltige Systeme haben jedoch toxikologische und ökologische Nachteile. Der hohe Gehalt an brennbaren und gesundheitsschädlichen Lösungsmitteln ist aus Gründen des Arbeits-, Gesundheits- und Umweltschutzes ungünstig. Zudem unterliegt der Einsatz von Lösungsmitteln zunehmend gesetzlichen Regulierungen. Diese ergeben sich unter anderem aus verschiedenen nationalen und internationalen Richtlinien (EU-Decopaint-Richtlinie) zur Begrenzung von VOC-Emissionen (engl.: volatile organic compound = deutsch: flüchtige organische Verbindungen) aus Beschichtungsstoffen und zur Reduzierung des Gesundheitsrisikos des Verarbeiters und Nutzers durch flüchtige und schwerflüchtige Verbindungen (VOC und SVOC) (siehe Forderungen des Ausschusses für die gesundheitliche Bewertung von Bauprodukten = AgBB) oder die Zertifizierung von Gebäuden gemäß der Deutschen Gesellschaft für Nachhaltiges Bauen e.V. (DGNB) oder der Leadership in Energy & Environmental Design (LEED).

Wasserbasierte Farben und Lacke werden daher in großem Maßstab industriell verwendet.

Es wäre daher wünschenswert, eine wasserbasierte härtbare Zusammensetzung der eingangs genannten Art zur Verfügung zu stellen, mit der wasserbasierte Beschichtungen hergestellt werden können, bei denen ein Schutz gegen Mikroorganismen bereitgestellt wird, wobei die anti-mikrobielle Wirkung auf physikalischem Weg erfolgen soll. Die härtbare Zusammensetzung würde somit nicht unter die Biozid-Verordnung (Verordnung (EU), Nr. 528/2012 des Europäischen Parlaments und des Rates vom 22. Mai 2012 in der aktuellen Fassung von 2019) fallen, was ein Verzicht von Zulassungsdauer und - kosten von großem Vorteil bedeutet.

Basierend auf der Lehre der Europäischen Patentanmeldungen EP 19202910.6, PCT/EP2020/077798 und EP 21157055.1 schlägt die vorliegende Erfindung vor, eine wasserbasierte härtbare Zusammensetzung zur Herstellung von Beschichtungen mit anti-mikrobieller Eigenschaft enthaltend
- mindestens ein filmbildendes Polymer,
- optional mindestens ein Additiv,
- optional mindestens einen Härter,
- mindestens einen up-conversion Leuchtstoff der allgemeinen Formel (I)

   A_{1-x-y-z}B*_{y}B₂SiO₄:Ln¹ₓLn²_{z}, I

   mit
   x = 0,0001 - 0,0500;
   z = 0,0000 oder z = 0,0001 bis 0,3000 mit der Maßgabe, dass gilt: y = x + z;
   A ausgewählt aus der Gruppe bestehend aus Mg, Ca, Sr und Ba;
   B ausgewählt aus der Gruppe bestehend aus Li, Na, K, Rb und Cs;
   B* ausgewählt aus der Gruppe bestehend aus Li, Na und K, wobei B gleich B* oder B ungleich B* ist, bevorzugt B und B* sind ungleich;
   Ln¹ ausgewählt aus der Gruppe bestehend aus Praseodym (Pr), Erbium (Er) und Neodym (Nd);
   Ln² ausgewählt aus Gadolinium (Gd),
   wobei der Leuchtstoff durch eine Nachbehandlung mindestens ein Material, das eine Bandlücke von größer als 6 Elektronenvolt (eV) hat und hydrolysestabil ist, aufweist.

Es wurde festgestellt, dass der Leuchtstoff, hergestellt nach der Lehre der Europäischen Patentanmeldungen EP 19202910.6, PCT/EP2020/077798 und EP 21157055.1, keine up-conversion mehr aufweist, wenn dieser in Wasser suspendiert wird.

Ohne an einer Theorie gebunden zu sein, wird vermutet, dass bestimmte Elemente des Leuchtstoffs in Lösung gehen und somit die Kristallgitterstruktur des Leuchtstoffs dermaßen gestört wird, dass der Leuchtstoff keine up-conversion mehr aufweist. Die physikalische anti-mikrobielle Wirkung könnte somit verloren gehen.

Mit dem nun nachbehandelten erfindungsgemäßen Leuchtstoff konnte überraschenderweise eine Diffusionsbarriere geschafften werden, so dass die erfindungsgemäße wasserbasierte härtbare Zusammensetzung für die Herstellung einer anti-mikrobiell wirkenden Beschichtung verwendet werden kann. Es wurde unerwartet festgestellt, dass der Leuchtstoff einerseits die für die anti-mikrobielle Wirkung erforderliche Wellenlänge emittieren kann und zudem hydrolysestabil ist.

Hierbei könnte die Bandlücke des bevorzugten Materials eine Rolle bei der Diffusionsbarriere spielen. So lässt das bevorzugte Material UV-C Strahlung (200 - 280 nm) ohne Abschwächung hindurch.

Vorzugsweise weist das Material eine Bandlücke nicht höher als 12 Elektronenvolt (eV) auf.

Vorzugsweise ist das Material ausgewählt aus der Gruppe bestehend aus Oxiden, Silikaten, Boraten, Phosphaten eines anorganischen Materials oder einer Mischung davon.

Bevorzugt ist das Material ausgewählt aus der Gruppe bestehend aus SiO₂, α-Al₂O₃, MgO, MgAl₂O₄, Ca-polyphosphate, Sr-polyphosphate, Ca- oder Sr-Pyrophosphat, (Ca₁₋ₓSrₓ)₃P₂O₇ (mit x = 0,0 - 1,0) oder einer Mischung davon.

Vorzugsweise wurde das Material durch eine Nachbehandlung mit einem Ausgangsstoff auf dem Leuchtstoff ausgebildet.

Als Ausgangsstoffe für die Nachbehandlung sind bevorzugt Tetraalkylorthosilikate, wobei die Alkylgruppen gleich oder verschieden bei jedem Auftreten 1 bis 10 C-Atome, bevorzugt gleich oder verschieden bei jedem Auftreten 1 bis 4 C-Atome aufweisen, insbesondere Tetramethyl-, Tetraethyl-, Tetra-n-propyl-, Tetra-iso-propyl- und Tetrabutylorthosilicat und/oder Mischungen davon.

Besonders bevorzugt ist Tetraethylorthosilikat (TEOS), Tetramethylorthosilikat (TMOS), Tetraisopropylorthosilikat (TiPOS), Tetrapropylorthosilikat (TPOS), Tetrabutylorthosilikat (TBOS) und/oder Mischung davon.

Auch bevorzugt als Ausgangsstoffe sind Al-Alkoholate, wie z. B. Al-methanolat, -ethanolat, -propanolat, -isopropanolat, -butanolat oder Mg-Alkoholate, wie z. B. Mg-methanolat, - ethanoat, -propanolat, -isopropanolat, -butanolat, oder AI- und Mg-Alkoholate, wie z. B. Al-/Mg-methanoat, -ethanolat, -propnolat, -isopropanolat, -butanolat, oder Ca-/Sr-Nitrate, - Acetate, -Oxalate und Natriumpolyphosphat, oder Natriumpyrophosphat, oder Mischung davon.

Vorzugsweise weist der Leuchtstoff nach der Nachbehandlung einen kristallinen Kern mit einer glasartigen oder amorphen Umhüllung auf.

Bevorzugt wurde der Leuchtstoff vor der Nachbehandlung mit mindestens einem Flussmittel hergestellt.

Aus dem Stand der Technik sind zahlreiche Flussmittel aller Art, wie Halogenide, Carbonate, Sulfate, Oxide und Borate von jeweils, wo zutreffend, Ammonium, Lithium, Natrium, Kalium, Rubidium, Cäsium, Magnesium, Calcium, Strontium, Barium, Blei, Lanthan, Lutetium, Aluminium, Bismut sowie Borsäure, dem Fachmann bekannt. Auch bekannt sind deren Einsatzgebiete im Bereich der Metallurgie, um beispielsweise das Kristallwachstum zu beschleunigen oder die Bildung von Fremdphasen zu unterbinden.

Das Auffinden geeigneter Flussmittel war daher auch von besonderer Bedeutung.

Völlig überraschend konnte festgestellt werden, dass die Herstellung der up-conversion Leuchtstoffe in Gegenwart von mindestens einem halogenhaltigen Flussmittel zu up-conversion Leuchtstoffe mit homogener Partikelgrößenverteilung sowie erhöhter Intensität der Emission bzw. höherer Quantenausbeute führen, im Vergleich zu Leuchtstoffen ohne Zusatz eines Flussmittels oder mit einem anderen Flussmittel.

Flussmittel, im englisch sprachigen Raum als "flux" übersetzt, ist auch unter dem Begriff Fluxmittel bekannt. Die Behandlung mit Flussmittel wird auch fluxen genannt bzw. das Produkt wurde gefluxt.

Es konnte in den Beispielen gezeigt werden, dass die Partikelgrößenverteilung einer Gauß'schen Verteilung ähnelt. Die Partikelgröße sind homogener, sodass deren Einarbeitung in einer Lackmatrix wesentlich leichter durchgeführt werden kann.

Es konnte festgestellt werden, dass die Partikelgrößen der erfindungsgemäßen Leuchtstoffe durch das Flussmittel homogener sind. Somit können diese leichter in die Lackmatrix eingearbeitet werden, was möglicherweise zu verbesserten Lackeigenschaften, wie etwa das Erscheinungsbild der Lackoberfläche, beispielsweise der Glanz, Haptik und Touch, führen können.

Auch konnte die Intensität der Emission der up-conversion Leuchtstoffe durch eine einfache technische Durchführung der Synthese erzielt werden.

Somit ist auch ein weiterer Gegenstand der Erfindung ein Verfahren zur Herstellung dieser up-conversion Leuchtstoffe sowie damit erhältliche up-conversion Leuchtstoffe.

Vorzugsweise wird als halogenhaltiges Flussmittel mindestens ein Stoff aus der Gruppe der Ammoniumhalogenide, Alkalimetallhalogenide, Erdalkalimetallhalogenide und Lanthanoidhalogenide eingesetzt. Halogenide dieser Gruppen haben überraschend gezeigt, dass damit hergestellte up-conversion Leuchtstoffe eine höhere Emission-Intensität aufweisen als mit anderen Flussmitteln.

Vorzugsweise handelt es sich bei den Halogeniden um Fluoride oder Chloride.

Bevorzugt handelt es sich bei den Alkalimetallen um Kalium, Natrium oder Lithium.

Bevorzugt handelt es sich bei dem Lanthanoid um Praseodym.

Bevorzugt handelt es sich bei den Erdalkalimetallen um Calcium oder Strontium.

Vorzugsweise ist der Leuchtstoff mit Praseodym dotiert, der in der erfindungsgemäßen Zusammensetzung verwendet wird.

Bevorzugt für die erfindungsgemäße Zusammensetzung ist der Leuchtstoff mit Praseodym dotiert und mit Gadolinium co-dotiert.

Es ist bevorzugt, dass der Leuchtstoff zumindest partiell kristallin ist. Es ist also bevorzugt, dass der Leuchtstoff partiell oder vollständig kristallin ist. Vorzugsweise ist der Leuchtstoff also zumindest nicht vollständig amorph. Es ist daher bevorzugt, dass der Leuchtstoff keine amorph erstarrte Schmelze (Glas) ist.

Vorzugsweise ist der Leuchtstoff eine erstarrte Schmelze aus kristallinen Silikaten oder aus kristallinen Silikaten, dotiert mit Lanthanoid-Ionen, umfassend mindestens einen Alkalimetallion und mindestens einen Erdalkalimetallion.

Bevorzugt für die erfindungsgemäße Zusammensetzung ist der Leuchtstoff ausgewählt aus Verbindungen der allgemeinen Formel (la)

A_{1-x-y-z}B*_{y}B₂SiO₄:Prₓ,Gd_{z}, (Ia)

mit A ausgewählt aus der Gruppe bestehend aus Mg, Ca, Sr, Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
x = 0,0001 - 0,0500;
z = 0,0000 oder z = 0,0001 bis 0,3000 mit der Maßgabe, dass gilt: y = x + z.
B* dient dabei zum Ladungsausgleich der Silikatanionen.

A kann dabei für ein einziges Element aus der Gruppe bestehend aus Mg, Ca, Sr und Ba stehen, oder auch für eine Kombination von zwei oder mehr Elementen dieser Gruppe, also z.B. A = (Mgₐ₁ Caₐ₂ Srₐ₃ Baₐ₄) mit 0 ≤ a1 ≤ 1, 0 ≤ a2 ≤ 1, 0 ≤ a3 ≤ 1 0≤ a4 ≤ 1 und mit der Maßgabe, dass gilt: a1+a2+a3+a4 = 1. A kann also z.B. für (Ca_{0,9}Sr_{0,1}) stehen.

Vorzugsweise wird für die erfindungsgemäße Zusammensetzung der Leuchtstoff ausgewählt aus Verbindungen der allgemeinen Formel (II)

(Ca₁₋ₐSrₐ)_{1-2b}Ln_{b}Na_{b}Li₂SiO₄ II

wobei gilt:
Ln ist ausgewählt aus der Gruppe bestehend aus Praseodym, Gadolinium, Erbium, Neodym, vorzugsweise Praseodym;
a = 0,0000 bis 1,0000, vorzugsweise 0,0000 bis 0,1000, insbesondere 0,0000;
b = 0,0001 bis 1,0000, vorzugsweise 0,0001 bis 0,1000, insbesondere 0,0050 bis 0,0150.

Ln kann dabei für ein einziges Element aus der Gruppe bestehend aus Praseodym, Gadolinium, Erbium und Neodym stehen, oder auch für eine Kombination von zwei Elementen dieser Gruppe, also z.B. Ln = (Ln¹ₓ Ln²_{y}), wobei Ln¹ und Ln² ausgewählt sind der Gruppe bestehend aus Praseodym, Gadolinium, Erbium und Neodym, und wobei x und y wie für Formel (I) und (Ia) definiert sind.

Ln¹ dient der Dotierung. Für die Dotierung wird vorzugsweise Praseodym verwendet. Ln² dient der optionalen Co-Dotierung. Für die optionale Co-Dotierung wird vorzugsweise Gadolinium verwendet. Vorzugsweise ist der Leuchtstoff nicht co-dotiert, d.h. Ln steht für vorzugsweise für ein einziges Element aus der Gruppe bestehend aus Praseodym, Gadolinium, Erbium und Neodym.

Es ist noch bevorzugter, dass der Leuchtstoff ausgewählt ist aus Verbindungen der allgemeinen Formel (IIa)

Ca_{1-2b}Pr_{b}Na_{b}Li₂SiO₄ (IIa)

mit b = 0,0001 bis 1, vorzugsweise 0,0001 bis 0,1, insbesondere 0,005 bis 0,015.

Es ist ganz besonders bevorzugt, dass der Leuchtstoff Ca_{0,98}Pr_{0,01}Na_{0,01}Li₂SiO₄ ist.

Bevorzugt weist der erfindungsgemäße up-conversion Leuchtstoff ein Halogen, entsprechend dem Halogenid des Flussmittels, auf.

Bevorzugt handelt es sich bei dem Leuchtstoff um einen, der bei Bestrahlung mit elektromagnetischer Strahlung mit geringerer Energie und höherer Wellenlänge im Bereich von 2000 bis 400 nm, insbesondere im Bereich von 800 bis 400 nm, elektromagnetische Strahlung mit höherer Energie und kürzerer Wellenlänge im Bereich von 400 bis 100 nm, bevorzugt im Bereich von 300 bis 200 nm emittiert. Es ist ferner bevorzugt, dass dabei die Intensität des Emissionsmaximums der elektromagnetischen Strahlung mit höherer Energie und kürzerer Wellenlänge eine Intensität von mindestens 1 • 10³counts/(mm²*s), bevorzugt höher als 1 • 10⁴ counts/(mm²*s), besonders bevorzugt höher als 1 • 10⁵ counts/(mm²*s). Zur Bestimmung dieser Kenngrößen wird die Emission bevorzugt mittels eines Lasers, insbesondere eines Lasers mit einer Leistung von 75 mW bei 445 nm und/oder einer Leistung von 150 mW bei 488 nm angeregt.

Vorzugsweise weist der Leuchtstoff gemäß Formel (II) XRPD Signale im Bereich von 23° 2Θ to 27° 2Θ und von 34° 2Θ bis 39.5° 2Θ auf, wobei die Signale mittels der Bragg-Brentano Geometrie und der Cu-K_{α} Radiation bestimmt werden. Einzelheiten der Messmethode können aus der noch unveröffentlichten europäischen Patentanmeldungen EP 19202910.6 und PCT/EP2020/077798 entnommen werden.

Die bisher unveröffentlichten europäischen Patentanmeldungen EP 19202910.6 und PCT/EP2020/077798 widmen sich der Herstellung von Leuchtstoffen, insbesondere von Leuchtstoffen gemäß Formel (I), Formel (la) und Formel (II), ohne den Zusatz von Flussmitteln und/oder den Ausgangsstoffen.

Ausgehend des darin beschriebenen Verfahrens umfasst das erfindungsgemäße Verfahren folgende Schritte:
- i) Bereitstellung mindestens eines Lanthanoidsalzes, ausgewählt aus Lanthanoidnitrate, Lanthanoidcarbonate, Lanthanoidcarboxylate, bevorzugt Lanthanoidacetate, Lanthanoidsulphate, Lanthanoidoxide, besonders bevorzugt Pr₆O₁₁ und/oder Gd₂O₃, wobei die Lanthanoidion in den Lanthanoidoxiden oder Lanthanoidsalzen ausgewählt ist aus Praseodymium, Gadolinium, Erbium, Neodym und für die Co-Dotierung mindestens zwei davon,
- ii) Bereitstellung eines Silikats, bevorzugt eines Silikatsalzes, besonders bevorzugt eines Alkalimetallsalzes des Silikats,
- iii) Bereitstellung mindestens eines Erdalkalimetallsalzes und mindestens eines Alkalimetallsalzes, bevorzugt eines Alkalimetallsilikats ausgewählt aus einem Lithiumsalz oder einer Lithiumverbindung und optional ausgewählt aus einem Natriumsalz und Kaliumsalz, vorzugsweise das Salz des Lithiumsalzes und ist ein Lithiumsilikat,
- iv) optional Bereitstellung mindestens eines Flussmittels aus der Gruppe der Ammoniumhalogenide, vorzugsweise Ammoniumchlorid, Alkalimetallhalogenide, vorzugsweise Natriumchlorid, Natriumfluorid, Natriumbromid, Lithiumfluorid, Lithiumchlorid, Erdalkalimetallhalogenide, vorzugsweise Calciumchlorid, Calciumfluorid und Lanthanoidhalogenide, vorzugsweise Praseodymfluorid, oder Praseodymchlorid,
- a) Mischen i), ii) iii) und optional iv) mittels Mahlens zur Erhaltung einer Mischung, oder
- b) Mischen i), ii) und iii) in einem organischen polaren oder unpolaren Lösemittel, das kein protisches Lösemittel ist, zur Erhaltung einer Mischung; die Mischung aus b) wird kalziniert (Schritt 1a) bei 600 bis 1000 °C zur Entfernung der organischen Komponente, bevorzugt wird die Kalzinierung bei 600 bis 1000 °C für mindestens 1 h durchgeführt, vorzugsweise mehr oder gleich 2 h, unter Standard (Luft)Atmosphäre zur Erhaltung einer kalzinierten Mischung,
- Kalzinieren der Mischung aus a) oder der kalzinierten Mischung aus b) in einem Kalzinierungsschritt, bevorzugt unter Luft bei einer Temperatur unterhalb der Schmelztemperatur des silikatbasierten Materials, wobei mindestens eine partielle Kristallisation stattfindet, bevorzugt in einem weiteren Kalzinierungsschritt (Schritt 1b) bei einer Temperatur von 50 bis 200 °C für mindestens 3h, vorzugsweise unter Luft, unterhalb der Schmelztemperatur des silikatbasierten Materials, um das silikatbasierte Material zu kristallisieren, bevorzugt bei einer Temperatur von 800 bis 900 °C, besonders bevorzugt bei ungefähr 850 °C, für mindestens 3 h, vorzugsweise für mindestens 12 h, vorzugweise unter Luft,
- in einem weiteren Kalzinierungsschritt bei steigender Temperatur, vorzugsweise über 800 °C und 50 bis 200 °C unterhalb des Schmelzpunktes (Schritt 2) des Materials, z.B. bei 850 °C für mindestens 3 h, besonders bevorzugt für mindestens 6 h, unter reduzierter Atmosphäre, dabei werden die Lanthanoide zu Ln³⁺ Ions reduziert,
- Erhalten eines silikatbasierten Lanthanoid-Ion dotierten Materials, bevorzugt nach Abkühlen des Materials,
- Nachbehandlung des silikatbasierten Lanthanoid-Ion dotierten Materials mit mindestens einem Ausgangsstoffes zur Erhaltung eines Materials mit einer Bandlücke von größer als 6,0 Elektronenvolt (eV) auf dem dotierten Material.

Weitere detaillierte Ausführungsformen des Verfahrens können aus der EP 19202910.6 und PCT/EP2020/077798 entnommen werden, wobei lediglich das Flussmittel zum Einsatz kommt mit anschließender Nachbehandlung mit einem Ausgangsstoff.

Vorzugsweise können 0,01 Gew.-% - 3,5 Gew.-%, bevorzugt 0,5 - 3,5 Gew.-%, besonders bevorzugt 1,0 - 3,5 Gew.-% Flussmittel, bezogen auf die Gesamtmenge der Edukte eingesetzt werden.

Es ist auch vorstellbar, den erfindungsgemäßen Leuchtstoff wie folgt herzustellen:
Als Ausgangsstoffe werden CaCO₃ (Fa. Alfa Aesar, 99.5%), Li₂CO₃ (Fa. Alfa Aesar, 99%), SiO₂ (Aerosil 200, Evonik), Pr₆O₁₁ (Treibacher, 99.99%), and Na₂CO₃ (Merck, 99.9%) sowie ein CaF₂ (Sigma Aldrich, 99.9 %) Flussmittel verwendet. Eine stöchiometrische Mischung dieser Verbindungen wird in Aceton für 30 Minuten vermischt. Nachdem das Aceton bei Raumtemperatur vollständig verdunstet ist, wird die Mischung in einem Korundtiegel überführt. Die Mischung wird zweimal kalziniert. Die erste Kalzinierung wird in einem Schmelzofen bei 850 °C für 12 h unter Luftzufuhr und die zweite Kalzinierung bei 850 °C für 6h unter 95/5 N₂/H₂ durchgeführt. Das Endprodukt wird in einer Achatreibschale anschließend gemahlen.

Der so hergestellte up-conversion Leuchtstoff kann anschließend vorzugsweise einer Nachbehandlung mit einem oben beschriebenen Ausgangsstoff unterzogen werden.

Es gibt viele Möglichkeiten die Nachbehandlung durchzuführen, so dass der Leuchtstoff einen im wesentlichen kristallinen Kern und eine glasartige bzw. amorphe Umhüllung bestehend aus dem oben beschriebenen Material erhält.

Ausgehend von dem oben beschriebenen Verfahren zur Herstellung des Leuchtstoffs umfasst die erfindungsgemäße Nachbehandlung vorzugsweise folgende Schritte:
- Dispergierung des Leuchtstoffs in einem wasserfreien Medium, vorzugsweise mit einem Alkohol, wie z. B. Ethanol, Methanol, Propanol, Butanol, iso-Propanol, isoButylalkohol oder Amylalkohole,
- Zugabe eines Ausgangsstoffes,
- Durchführen des Sol-Gel Prozesses im alkalischen Bereich, bevorzugt nach der Stöber-Synthese, einem anderen Sol-Gel Verfahren oder einer homogenen Präzipitation mit Harnstoff, Urotropin oder einem anderen Hydroxidionen-Lieferanten.
- Optional Zugabe eines weiteren Ausgangsstoffes,
- Deagglomeration, vorzugsweise mittels Ultraschalles oder durch Rühren mit Mahlkörpern oder durch Mikrofluidisierung,
- Entfernung des wasserfreien Mediums und
- Trocknung des Leuchtstoffes.

Vorzugsweise kann die Zugabe des Ausgangsstoffes tropfenweise oder sprühartig auf den Leuchtstoff aufgebracht werden.

Deagglomeration kann vorzugsweise auch mittels Rotor-Stator-Systeme, beispielweise mit Rührwerken, Kolloidmühle, Homogenizer oder mittels Spray Drying erfolgen.

In einer bevorzugten Ausführung wird die Nachbehandlung mit einer Mischung aus Tetraethyl-ortho-silikat (TEOS) und Tetra-methyl-ortho-silicate (TMOS) durchgeführt.

Es ist auch vorstellbar, die Nachbehandlungsschritte zu optimieren, indem ein Sprühen des Ausgangsstoffes auf den Leuchtstoff mittels Wirbelschicht, Intensivmischer oder Innojet Verfahren durchgeführt wird.

Es hat sich überraschend herausgestellt, dass die Leuchtstoffe gemäß der EP 19202910.6 und PCT/EP2020/077798 nach der erfindungsgemäßen Nachbehandlung die erforderlichen up-conversion Eigenschaften in einem wässerigen Medium aufweisen, die für die antimikrobielle Wirkung verantwortlich sind. D. h. diese Leuchtstoffe können elektromagnetische Strahlung mit Wellenlängen oberhalb der UV-Strahlung, insbesondere sichtbares Licht oder Infrarotlicht, in elektromagnetische Strahlung mit geringerer Wellenlänge emittieren und zwar in dem Bereich, bei dem z.B. die DNA bzw. RNA der Mikroorganismen zerstört bzw. mutiert werden kann. Demnach sind diese Leuchtstoffe für die erfindungsgemäße Zusammensetzung sehr gut geeignet.

Ein weiterer Gegenstand ist ein Leuchtstoff nach einer Formel (I), (Ia), (II) oder (IIa), wobei der Leuchtstoff einen im wesentlichen kristallinen Kern mit einer glasartigen bzw. amorphen Umhüllung aufweist.

Vorzugsweise weist der Leuchtstoff eine glasartige Umhüllung aus einem Material mit einer Bandlücke von größer als 6 Elektronenvolt (eV) auf.

Vorzugsweise weist der Leuchtstoff eine glasartige Umhüllung aus einem Material mit einer Bandlücke von kleiner als 12 Elektronenvolt (eV) auf.

Bevorzugt weist der Leuchtstoff ein Material ausgewählt aus der Gruppe bestehend aus Oxiden, Silikaten, Boraten, Phosphaten eines anorganischen Materials oder einer Mischung davon auf.

Bevorzugt ist das Material ausgewählt aus der Gruppe bestehend aus SiO₂, α-Al₂O₃, MgO, MgAl₂O₄, Ca-polyphosphate, Sr-polyphosphate, Ca- oder Sr-pyrophosphat (Ca₁₋ₓSrₓ)₃P₂O₇ (mit x = 0,0 - 1,0) oder einer Mischung davon.

Vorzugsweise wurde der Leuchtstoff mit mindestens einem oben beschriebenen Flussmittel hergestellt und anschließend nachbehandelt, so dass der Leuchtstoff hydrolysestabil ist. Eine weitere Aufgabe der Erfindung ist die Auswahl filmbildender Polymere, die für die wässrige härtbare Zusammensetzung mit antimikrobieller Eigenschaft eingesetzt werden können. Grundsätzlich kommen alle aus dem Stand der Technik bekannten filmbildenden Polymere in Frage.

Vorzugsweise weist das filmbildende Polymer funktionelle Gruppen, vorzugsweise acide Wasserstoffe, auf, welche mit einem isocyanathaltigen Härter reaktiv sind und ggf. mit einem Katalysator katalysiert wird.

Unter wasserbasiert wird auch solche härtbaren Zusammensetzungen verstanden, die mit Wasser verdünnbar oder in Wasser löslich sind.

Vorteilhafterweise ist das filmbildende Polymer ausgewählt aus der Gruppe der hydroxyfunktionellen Acrylatpolymere, hydroxyfunktionellen Polyesterpolymere, und/oder hydroxyfunktionellen Polyetherpolymere, hydroxyfunktionellen Cellulosederivate, aminofunktionellen Polyesterpolymere oder Mischung davon, das mit einem isocyanathaltigen Härter reagiert.

Vorzugsweise werden diese filmbildende Polymere in Wasser unter Zuhilfenahme eines geeigneten Emulgators emulgiert oder gelöst. Dem Fachmann sind geeignete anionische, kationische und nicht-ionische Emulgatoren bekannt.

Vorzugsweise ist das filmbildende Polymer resonanzarm.

Dem Fachmann sind die physikalischen Wechselwirkungen an der Oberfläche bekannt. Je nach Material und dessen Materialoberfläche treten bei Lichteinfall eine Vielzahl von Effekten an der Oberfläche auf. Das auftreffende Licht wird zum Teil absorbiert, ein Teil wird reflektiert und je nach Materialoberfläche auch gestreut. Licht kann auch erst absorbiert und dann wieder emittiert werden. Bei opaken, halbdurchlässigen oder lichtdurchlässigen Materialien kann das Licht auch durch den Körper durchdringen (Transmission). In einigen Fällen wird das Licht an der Oberfläche sogar polarisiert oder gebeugt. Manche Objekte können sogar Licht emittieren (beleuchtete Anzeigen, LED-Segmente, Bildschirme), in einer anderen Lichtfarbe fluoreszieren oder phosphoreszieren (nachleuchten). Resonanzarm bedeutet im Sinne dieser Erfindung, dass das filmbildende Polymer geringe Absorption, Reflexion, Remission und Streuung im UV-Bereich aufweist. Dagegen soll vorzugsweise die Transmission ausgeprägt sein.

Es konnte nämlich überraschend festgestellt werden, dass die erfindungsgemäße filmbildende Polymere, die resonanzarm sind, eine verbesserte antimikrobielle Wirkung aufweisen, bedingt dadurch, dass mehr elektromagnetische Strahlung mit geringerer Energie und höherer Wellenlänge im Bereich von 2000 nm bis 400 nm, insbesondere im Bereich von 800 nm bis 400 nm, transmittiert wird und resultierend daraus mehr elektromagnetische Strahlung mit höherer Energie und kürzerer Wellenlänge im Bereich von 400 nm bis 100 nm, bevorzugt im Bereich von 300 nm bis 200 nm, emittieren kann.

Es wurde festgestellt, dass je höher die Transmission ist, desto höher ist auch die Emission, die für die antimikrobielle Wirkung ausschlaggebend ist.

Vorzugsweise liegt die Transmission des filmbildenden Polymers bei mindestens 75%, bevorzugt mindestens 80% und besonders bevorzugt mindestens 85%, gemessen bei einer Wellenlänge von 260 nm.

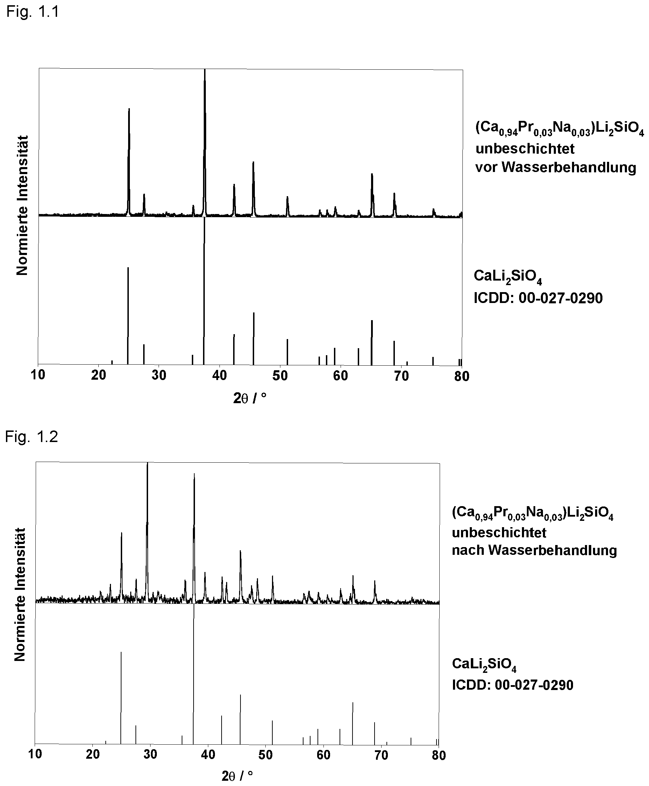

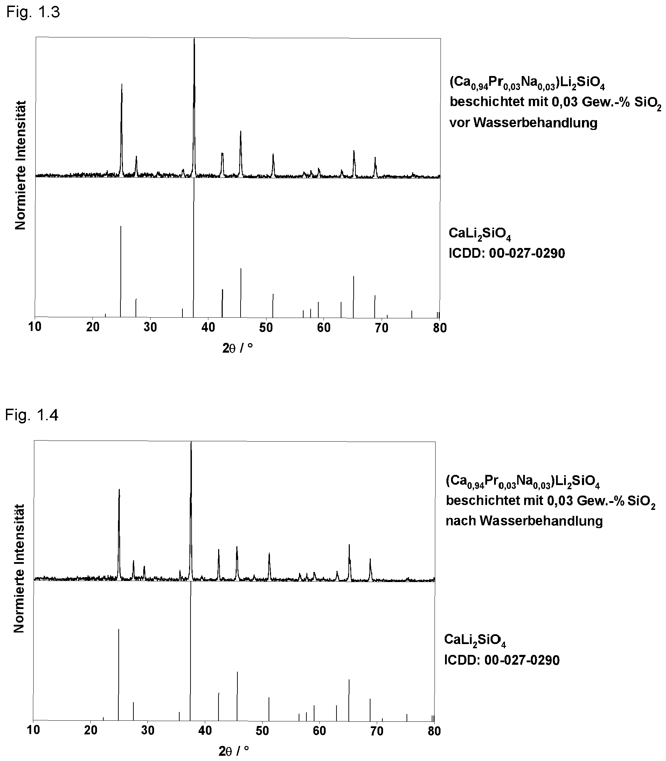

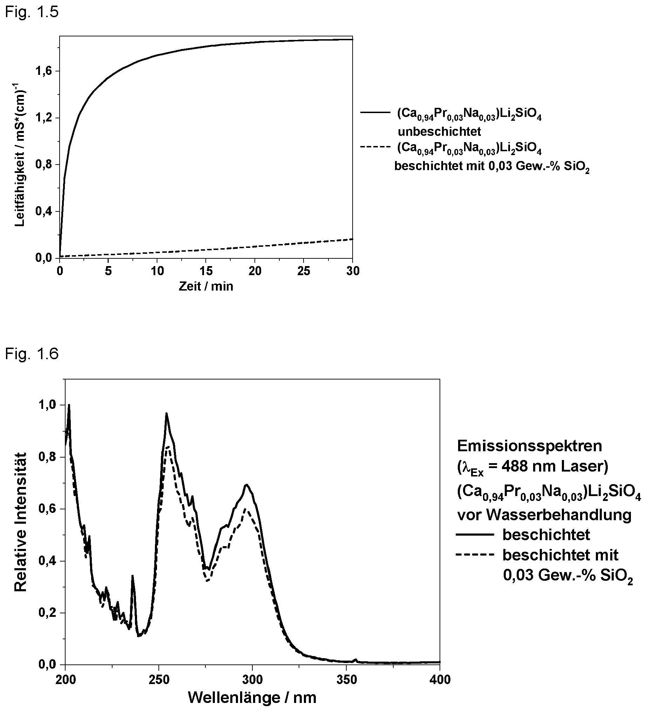

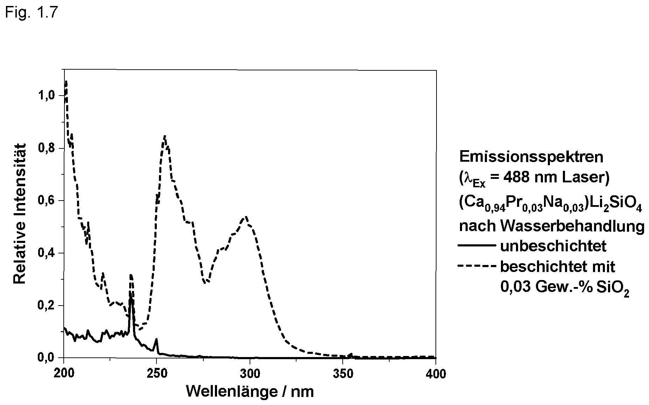

Vorzugsweise liegt die Transmission des filmbildenden Polymers bei mindestens 75%, bevorzugt mindestens 80% und besonders bevorzugt mindestens 85%, gemessen bei einer Wellenlänge von 500 nm.
Zur Verdeutlichung sei hier angemerkt, dass die Transmission bei einer anderen Wellenlänge definiert werden kann, siehe Abbildung 1. Für die vorliegende Erfindung wurden die Wellenlängen 260 nm beispielshaft für die emittierte Wellenlänge und 500 nm beispielhaft für die Anregungswellenlänge gewählt, die zum einen für die up-conversion und zum anderen signifikant für die antimikrobielle Wirkung verantwortlich sind.

Bei einer Transmission von 100% beispielsweise, gemessen bei einer Wellenlänge 260 nm, wird die gleiche Menge an Strahlung umgewandelt und emittiert, d. h. es gibt keine Verluste durch Absorption, Streuungen oder dergleichen. Bei einer Transmission von 80%, gemessen bei einer Wellenlänge 260 nm, transmittiert 20% nicht, vermutlich aufgrund von Absorption, Reflexion, Remission und/oder Streuung. Demzufolge kann nur 80% der Strahlung der Wellenlänge 260 nm emittiert werden.

Diese bedeutende Erkenntnis ist wichtig bei der Auswahl der filmbildende Polymere. Polymeren, die z.B. 0% Transmission aufweisen, eignen sich nicht für die erfindungsgemäße härtbare Zusammensetzung. Sie transmittieren keine elektromagnetische Strahlung mit geringerer Energie und höherer Wellenlänge und demzufolge können in der Zusammensetzung enthaltene Leuchtstoffe diese elektromagnetische Strahlung nicht in elektromagnetische Strahlung mit höherer Energie und kürzerer Wellenlänge umwandeln und emittieren, die für die antimikrobielle Wirkung erforderlich ist.

Vorzugsweise weist die erfindungsgemäße Zusammensetzung eine Transmission bei mindestens 75%, bevorzugt bei mindestens 80% und besonders bevorzugt bei mindestens 85% gemessen bei 260 nm beträgt.

Vorzugsweise weist die erfindungsgemäße Zusammensetzung eine Transmission bei mindestens 75%, bevorzugt bei mindestens 80% und besonders bevorzugt bei mindestens 85% gemessen bei 500 nm beträgt.

Die Transmissionskurven werden vorzugsweise mit einem "Specord 200 Plus" UV/VIS-Zweistrahlspektrometer der Firma Analytik Jena gemessen. Mit einem Holmiumoxidfilter erfolgt eine interne Wellenlängenkalibrierung. Die Proben werden mit monochromatisiertem Licht einer Deuterium- (UV-Bereich) oder einer Wolfram-Halogen-Lampe (sichtbarer Bereich) durchstrahlt. Die spektrale Bandbreite beträgt 1,4 nm. Das monochromatisierte Licht wird in einen Mess- und einen Referenzkanal aufgeteilt und ermöglicht das direkte Messen gegen eine Referenzprobe. Die durch die Probe transmittierende Strahlung wird von einer Photodiode detektiert und zu elektrischen Signalen verarbeitet.

Es ist vorstellbar, eine Zusammensetzung mit einer geringen Transmission von unter 70 % einzusetzen; sie wirken möglicherweise auch noch antimikrobiell, jedoch ist der Wirkungsgrad sehr moderat.

Vorzugsweise weisen die Leuchtstoffe eine durchschnittliche Partikelgröße von d50 von 0,1 - 50 µm, bevorzugt d50 = 0,1 - 25 µm, besonders bevorzugt d50 = 0,1 - 5 µm, gemessen nach ISO 13320:2020 und USP 429, beispielsweise mit einem Gerät der Fa. Horiba, LA-950 Laser Particle Size Analyzer, auf.

Um die Leuchtstoffe in der erfindungsgemäßen Zusammensetzung gut einzubinden und/oder zu stabilisieren, können vorzugsweise verschiedene Additive zugesetzt werden.

Bevorzugt sind die Additive ausgewählt aus der Gruppe der Dispergiermittel, Rheologiehilfsmittel, Verlaufsmittel, Netzmittel, Entschäumer und UV-Stabilisierungsmittel.

Es wurde überraschend festgestellt, dass durch etwaigen Zusatz an Additiven in die erfindungsgemäße Zusammensetzung die Transmission reduziert wird.

Demnach weist die erfindungsgemäße Zusammensetzung in einer weiteren Ausführungsform, in der Additive eingesetzt werden, vorzugsweise eine Transmission bei mindestens 70%, bevorzugt bei mindestens 75% und besonders bevorzugt bei mindestens 80% gemessen bei 260 nm beträgt.

Demnach weist die erfindungsgemäße Zusammensetzung in einer weiteren Ausführungsform, in der Additive eingesetzt werden, vorzugsweise eine Transmission bei mindestens 70%, bevorzugt bei mindestens 75% und besonders bevorzugt bei mindestens 80% gemessen bei 500 nm beträgt.

Vorzugsweise weist die erfindungsgemäße Zusammensetzung einen Härter auf, der ausgewählt ist aus der Gruppe der aliphatischen oder cycloaliphatischen Isocyanate oder Mischung daraus.

Beispiele für isocyanathaltige Härter sind monomere Isocyanate, polymere Isocyanate und Isocyanat-Prepolymere. Polyisocyanate werden gegenüber monomeren Isocyanaten aufgrund ihrer geringeren Toxizität bevorzugt. Beispiele für Polyisocyanate sind Isocyanurate, Uretdione und Buirete basierend auf Diphenylmethan Diisocyanat (MDI), Toluol Diisocyanat (TDI), Hexamethylen Diisocyanate (HDI) and Isophoron Diisocyanat (IPDI). Beispiele für kommerziell erhältliche Produkte sind unter dem Markennamen DESMODUR^{®} von Covestro oder VESTANAT von der Evonik Industries. Bekannte Produkte sind DESMODUR^{®} N3400, DESMODUR^{®} N3300, DESMODUR^{®} N3600 DESMODUR^{®} N75, DESMODUR^{®} XP2580, DESMODUR^{®} Z4470, DESMODUR^{®} XP2565 and DESMODUR^{®} VL, von Covestro. Weitere Beispiele sind VESTANAT^{®} HAT 2500 LV, VESTANAT^{®} HB 2640 LV oder VESTANAT^{®} T 1890E von der Evonik Industries. Beispiele für Isocyanat-Prepolymere sind DESMODUR^{®} E XP 2863, DESMODUR^{®} XP 2599 oder DESMODUR^{®} XP 2406 von Covestro. Weitere dem Fachmann bekannte Isocyanat-Prepolymere können eingesetzt werden. Ganz besonders bevorzugt sind hydrophilierte Isocyanate wie Bayhydur 3100 von Covestro.

Es ist vorstellbar, Katalysatoren zur Aushärtung einzusetzen. Folgende Katalysatoren ausgewählt aus organischen Sn(IV)-, Sn(II)-, Zn-, Bi-Verbindungen oder tertiären Aminen können eingesetzt werden.

Bevorzugt werden Katalysatoren ausgewählt aus der Gruppe der Organozinnkatalysatoren, zyklische Amidine, Guanidine oder Amine oder eine Mischung daraus eingesetzt.

Der Härtungskatalysator wird vorzugsweise in Mengen von 0,01 bis 5,0 Gew.-%, bevorzugt 0,05 bis 4,0 Gew.-% und besonders bevorzugt 0,1 bis 3 Gew.-% bezogen auf das Gesamtgewicht der härtbaren Zusammensetzung eingesetzt.

Bei filmbildenden Polymeren, die durch physikalische Trocknung aushärten, ist die Zugabe von reaktiven Härtern nicht erforderlich.

Die erfindungsgemäße Zusammensetzung können vorzugsweise in 1K-Beschichtungssysteme oder 2K- Beschichtungssysteme, in Melamin Einbrenn-Systeme, Raum- oder Hochtemperatursysteme eingesetzt werden.

Vorzugsweise weisen aus der erfindungsgemäßen Zusammensetzung hergestellte Beschichtungen eine antimikrobielle Wirkung gegen Bakterien, Hefen, Schimmelpilze, Algen, Parasiten und Viren auf.

Bevorzugt weisen die erfindungsgemäßen hergestellten Beschichtungen eine antimikrobielle Wirkung gegen
- Erreger nosokomialer Infektionen, vorzugsweise gegen *Enterococcus faecium, Staphylococcus aureus, Klebsiella pneumoniae, Acinetobacter baumannii, Pseudomonas aeruginosa, Escherichia coli, Enterobacter, Corynebacterium diphteria, Candida albicans,* Rotavirus, Bakteriophagen;
- Fakultativ pathogene Umweltorganismen, vorzugsweise gegen *Cryptosporidium parvum, Giardia lamblia,* Amöben *(Arcanthamoeba* spp., *Naegleria* spp.), *E. coli,* coliforme Bakterien, Fäkalstreptokokken, *Salmonella* spp., *Shigella* spp., *Leginonella* spec., *Pseudomonas aeruginosa, Mykobakteria* spp., enterale Viren (z.B. Polio- und Hepatitis-A Virus);
- Pathogene in Lebensmitteln, vorzugsweise gegen *Bacillus cereus, Campylobacter* spp., *Clostridium botulinum, Clostridium perfringens, Cronobacter* spp., *E. coli, Listeria monocytogenes, Salmonella* spp., *Staphylococcus aureus, Vibrio* spp., *Yersinia enterocolitica,* Bakteriophagen,
   auf.

Es wurde festgestellt, dass die Einarbeitung der erfindungsgemäßen up-conversion Leuchtstoffe deutlich verbessert wurde.

Up-conversion Leuchtstoffe und Leuchtstoffe werden als Synonyme verwendet.

Ein weiterer Gegenstand ist die Verwendung der erfindungsgemäßen Zusammensetzung zur Herstellung von Dispersionen, Mahlgütern, Klebstoffen, Spachtelmassen, Putzen, Farben, Lacken oder Drucktinten, Inkjets, Anreibeharzen oder Pigmentkonzentraten.

Vorzugsweise ist die Verwendung der erfindungsgemäßen Zusammensetzung zur Herstellung von Beschichtungen mit anti-mikrobieller Eigenschaft.

Eine Beschichtung mit antimikrobieller Wirkung oder antimikrobieller Eigenschaft bedeutet hierbei, dass die Beschichtung eine antimikrobielle Oberfläche aufweist, die das Wachstum und die Vermehrung von Mikroorganismen begrenzt oder verhindert.

Es wurde erstaunlicherweise auch festgestellt, dass die erfindungsgemäßen Beschichtungen eine chemische und mechanische Beständigkeit aufweisen. Die chemische und mechanische Beständigkeit ist besonders bedeutsam, da antimikrobielle Beschichtungen häufig in Bereichen eingesetzt werden, die eine regelmäßige Desinfektion und weitere Hygienemaßnahmen erfordern.

Auch zur Erfindung gehört ein Verfahren zur Bildung einer anti-mikrobiellen Beschichtung auf einem Substrat, umfassend das Aufbringen einer härtbaren filmbildenden Zusammensetzung auf das Substrat, umfassend:
a. mindestens ein filmbildendes Polymer, das funktionelle Gruppen enthält, welche mit einem isocyanathaltigen Härter reaktiv sind, ggf. durch einen Katalysator katalysiert,
b. mindestens einen Leuchtstoff gemäß der Formel (II) und
c. einen Härter, enthaltend isocyanatfunktionelle Gruppen.

Vorzugsweise handelt es sich bei dem Substrat um Metall, mineralische Substrate (wie etwa Beton, Naturstein oder Glas), zellulosehaltige Untergründe, Holz sowie deren Hybride, formstabile Kunststoffe und/oder Duromere.

Unter dem Begriff "formstabile Kunststoffe" werden folgende, jedoch nicht abschließende, Polymere verstanden: Acrylnitril-Butadien-Styrol (ABS), Polyamide (PA), Polylactat (PLA), Polymethylmethacrylat (PMMA), Polycarbonat (PC), Polyethylenterephthalat (PET), Polystyrol (PS), Polyetheretherketon PEEK), Polyvinylchlorid (PVC). Polypropylen (PP), Polyethylen (PE).

Bevorzugt kann auf das Substrat vor der Auftragung der härtbaren filmbildenden Zusammensetzung eine Grundierzusammensetzung aufgetragen werden.

Vorzugsweise wird die erfindungsgemäße härtbare Zusammensetzung zur Beschichtung von Substraten in Hygieneeinrichtungen, Krankenhäusern und in der Lebensmittelindustrie eingesetzt.

Es können alle Bereiche des öffentlichen Raums, wie z.B. Schule, Altenheim, Großküche oder Kindergarten in Frage kommen.

Eine weitere Erfindung ist der Gegenstand, der mindestens teilweise, vorzugsweise vollständig, mit der erfindungsgemäßen härtbaren Zusammensetzung beschichtet ist.

Es sei hier anzumerken, dass die Begrifflichkeiten "antimikrobieller Effekt", antimikrobielle Wirksamkeit", "antimikrobielle Wirkung" und "antimikrobielle Eigenschaft" als Synonyme verwendet werden.

Es sei hier anzumerken, dass der erfindungsgemäße Gegenstand vorzugsweise auch ohne Freisetzung eines antimikrobiellen Wirkstoffs eine antimikrobielle Wirkung aufweisen kann, wenn die Beschichtung spezielle Leuchtstoffe, wie in den Ansprüchen beschrieben, enthalten. Auf diese Weise wird ein physikalischer Weg beschritten, auf dem die Mikroorganismen dann abgetötet werden. Daher fallen solche Materialien nicht unter die Biozid-Verordnung (Verordnung (EU), Nr. 528/2012 des Europäischen Parlaments und des Rates vom 22. Mai 2012 in der aktuellen Fassung von 2019).

Nachfolgend werden Beispiele aufgeführt, die dem Fachmann allein zur Erläuterung dieser Erfindung dienen und stellen keinerlei Beschränkung des beanspruchten Gegenstandes dar.

### Methoden

Die **Rasterlelektronenmikroskopie** wurde mit einem Rasterelektronenmikroskop des Typs EVO MA 10 der Firma Zeiss durchgeführt. Das Rasterelektronenmikroskop wurde mit einer LaB₆-Kathode und einer Beschleunigungsspannung von 10 kV bei 2 pA betrieben. Vor der Messung wurde die Probenkammer auf einen Wert von ca. 5*10⁻⁹ mbar evakuiert. Der Topographiekontrast wurde durch Detektion der Sekundärelektronen ausgewertet. Die maximale Auflösung betrug 10 nm.

**Pulver-XRDP:** Die Röntgenpulverdiffraktogramme der Proben wurden mit einem Panalytical X'Pert PRO MPD Diffractometer, das in der Bragg-Brentano Geometrie arbeitet, aufgenommen, wobei Cu-K_{α} Strahlung und ein Linescan CCD Detektor verwendet wurde. Die Integrationszeit betrug 20 s, während die Schrittweite 0.017° betrug.

**Die Emissionsspektren** wurden mit Hilfe eines Edinburgh Instruments FLS920 Spektrometers, das mit einem 488 nm continuous-wave OBIS Laser der Firma Coherent sowie einem Peltier gekühlten (-20 °C) single-photon counting photomultiplier der Firma Hamamatsu (R2658P) ausgerüstet ist. Kantenfilters wurden zur Unterdrückung der Reflexe zweiter und höherer Ordnung, welche durch die Monochromatoren verursacht werden, verwendet.

**Die Leitfähigkeit** wurde mit einem Labor-Konduktometer 703 der Firma Knick ermittelt. Dazu wurden 0,1 g der Probe in 200 mL Wasser bei 300 rpm und Raumtemperatur dispergiert. Nach Eintauchen der Messelektrode in die Dispersion erfolgte die Messung der Leitfähigkeit über einen Zeitraum von 30 min, wobei alle 30 s ein Messwert aufgezeichnet wurde. Im Anschluss wurde die Probe abfiltriert und bei 150 °C über Nacht im Trockenschrank getrocknet für weitere Messungen, wie z. b. XRD und Emission.

### Beispiel 1 Herstellung eines Leuchtstoffes (Ca_{0.94}Pr_{0.03}Na_{0.03})Li₂SiO₄

2,8225 g (28,2 mmol) CaCO₃, 2,2167 g (30,0 mmol) Li₂CO₃, 1,8025 g (30,0 mmol) SiO₂, 0,0477 g (0,45 mmol) Na₂CO₃ und 0,1781 g (0,9 mmol) PrF₃ wurden in einer Achatreibschale in Aceton vermischt. Diese Mischung wurde bei 850 °C für 12h in Luft zur Entfernung von organischen Bestandteilen kalziniert. Anschließend wird die Kalzinierung bei 850 °C für weitere 6 h in Formiergasatmosphäre (5% H₂/95% N₂) durchgeführt, was zum gewünschten Produkt führt. Der Leuchtstoff wurde für weitere Messungen entnommen.

### Beispiel 1.1 Erfindungsgemäßer Leuchtstoff Nachbehandlung des Leuchtstoffes (Ca_{0.94}Pr_{0.03}Na_{0.03})Li₂SiO₄ mit 0,03 Gew.-% SiO₂

15 g des Leuchtstoffes wurde in 300ml getrocknetem Ethanol für 30 Minuten im Ultraschallbad suspendiert und anschließend dekantiert, um die feste Phase zu erhalten. Dieses Prozedere wurde zweimal durchgeführt. Der Leuchtstoff wurde dann in 360 mL getrockneten Ethanol gegeben, 0.6 mL TMOS wurde hinzugegeben. Nach 10 Minuten in Ultraschallbad wurde 45 mL konz. NH₃ unter Rühren dazugegeben. Anschließend wurde tropfenweise eine TEOS/ETOH-Mischung, bestehend aus 20 mL TEOS und 60 mL Ethanol, innerhalb von 1h dazugegeben. Während dieser Stunde wurde der Ultraschall alle 10 min für 10 s aktiviert. Diese Dispersion wurde dann 3h gerührt und der Ultraschall alle 15 min für 10 s aktiviert. Die feste Phase wurde abfiltriert und mit getr. Ethanol gewaschen. Anschließend über Nacht bei 200 °C getrocknet.

Fig. 1.1 zeigt ein Röntgenpulverdiffraktogramm (X-ray diffraction pattern) des Leuchtstoffes aus Beispiel 1 (oberes Diagramm) im Vergleich zu einem Referenz-Leuchtstoff (unteres normiertes Röntgenpulverdiffraktogramm). Es konnte gezeigt werden, dass der gewünschte Leuchtstoff mit gewünschter Dotierung hergestellt wurde.

Fig. 1.2 zeigt ein Röntgenpulverdiffraktogramm (X-ray diffraction pattern) des Leuchtstoffes aus Beispiel 1 (oberes Diagramm), wobei dieser in Wasser suspendiert wurde. Es zeigte sich, dass der Leuchtstoff sich verändert hat. Es wird vermutet, dass bestimmte Elemente sich aus der Kristallgitterstruktur herausgelöst haben.

Fig. 1.1 und Fig 1.2 zeigen die Instabilität des nicht-nachbehandelten Leuchtstoffes in Wasser.

Fig. 1.3 zeigt ein Röntgenpulverdiffraktogramm (X-ray diffraction pattern) des Leuchtstoffes aus Beispiel 1.1 (oberes Diagramm) im Vergleich zu einem Referenz-Leuchtstoff (unteres normiertes Röntgenpulverdiffraktogramm). Es konnte gezeigt werden, dass der gewünschte Leuchtstoff mit gewünschter Dotierung hergestellt wurde.

Fig. 1.4 zeigt ein Röntgenpulverdiffraktogramm (engl.: X-ray diffraction pattern) des Leuchtstoffes aus Beispiel 1.1 (oberes Diagramm), wobei dieser in Wasser suspendiert wurde. Es zeigte sich, dass der Leuchtstoff sich nicht verändert hat.

Fig. 1.5 zeigt die Leitfähigkeit des nachbehandelten und nicht-nachbehandelten Leuchtstoffes, der vorher in Wasser suspendiert wurde. Es ist ersichtlich, dass der nachbehandelte Leuchtstoff so gut wie keine Leitfähigkeit aufwies. Der erfindungsgemäße Leuchtstoff ist daher hydrolysestabil.

Fig. 1.6 zeigt ein Emissionsspektrum des Beispiels 1.1 (gestrichelte Linie) im Vergleich zu einem Vergleichs-Leuchtstoff des Beispiels 1. Die Messung wurde, bevor der Leuchtstoff in Wasser suspendiert wurde, durchgeführt. Das Spektrum zeigt deutlich, dass die Intensität beider Leuchtstoffproben im gewünschten Wellenlängenbereich liegen. Durch die Nachbehandlung verliert der erfindungsgemäße Leuchtstoff kaum an Intensität.

Fig. 1.7 zeigt ein Emissionsspektrum des Beispiels 1.1 (gestrichelte Linie) im Vergleich zu einem Vergleichs-Leuchtstoff des Beispiels 1, nachdem diese in Wasser suspendiert wurden. Es zeigte deutlich, dass der nicht-nachbehandelte Leuchtstoff keine up-conversion mehr aufweist, so dass er keine physikalische anti-mikrobielle Wirkung mehr aufweisen würde. Der erfindungsgemäße Leuchtstoff kann in einer erfindungsgemäßen wasserbasierten Zusammensetzung eingesetzt werden, mit der Beschichtungen mit anti-mikrobieller Wirkung hergestellt werden kann.

### Beispiel 2 Erfindungsgemäßer Leuchtstoff Nachbehandlung des Leuchtstoffes (Ca_{0.94}Pr_{0.03}Na_{0.03})Li₂SiO₄ mit 0,06 Gew.-% SiO₂

Die Nachbehandlung wurde analog wie Beispiel 1.1 durchgeführt, wobei die TEOS/ETOH-Mischung, bestehend aus 40 mL TEOS und 60 mL Ethanol, eingesetzt wurde.

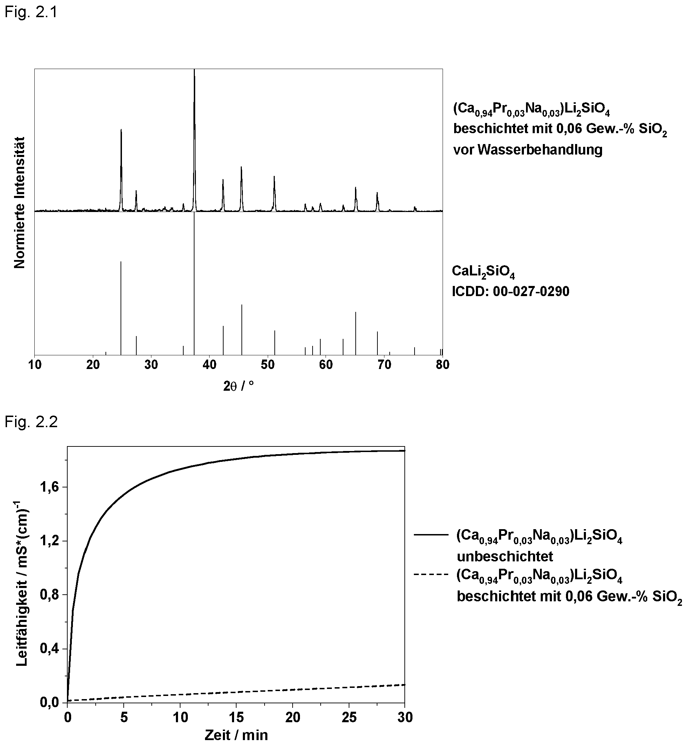

Fig. 2.1 zeigt ein Röntgenpulverdiffraktogramm (X-ray diffraction pattern) des Leuchtstoffes aus Beispiel 2 (oberes Diagramm) im Vergleich zu einem Referenz-Leuchtstoff (unteres normiertes Röntgenpulverdiffraktogramm). Es konnte gezeigt werden, dass der gewünschte Leuchtstoff mit gewünschter Dotierung hergestellt wurde.

Fig. 2.2 zeigt die Leitfähigkeit des nachbehandelten Leuchtstoffes gemäß Beispiel 2 und nicht-nachbehandeltes Leuchtstoffes, die vorher in Wasser suspendiert waren. Es ist ersichtlich, dass der nachbehandelte Leuchtstoff so gut wie keine Leitfähigkeit aufwies. Der erfindungsgemäße Leuchtstoff ist somit hydrolysestabil.

### Vergleichsbeispiel

### Beispiel 3: Nachbehandlung des Leuchtstoffes (Ca_{0.94}Pr_{0.03}Na_{0.03})Li₂SiO₄ mit 5 Gew.-% Y₂O₃

1 g (Ca_{0.94}Pr_{0.03}Na_{0.03})Li₂SiO₄ wurde mit 0.05 g Y₂O₃ in einer Achatschale vermischt.

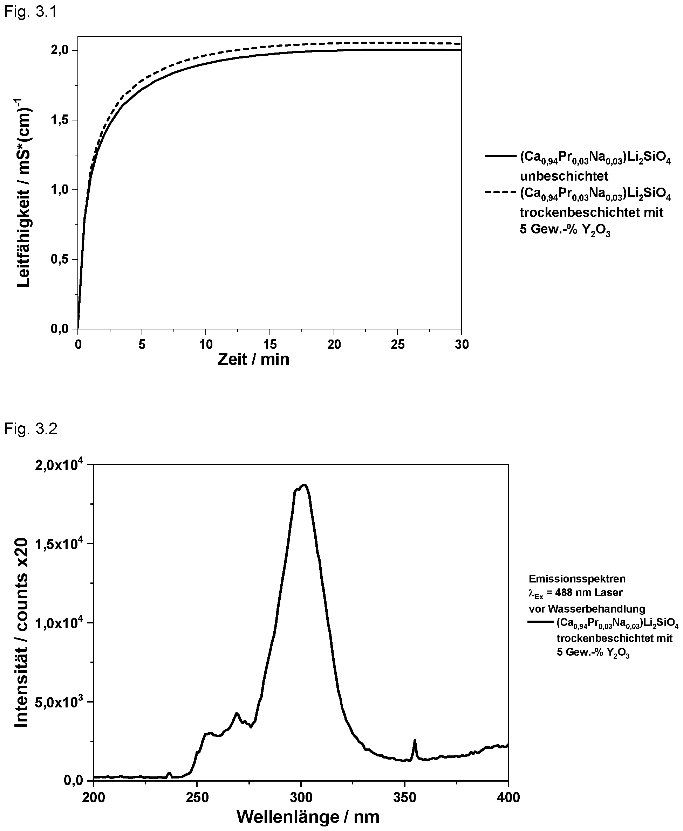

Fig. 3.1 zeigt die Leitfähigkeit des nachbehandelten Leuchtstoffes gemäß Beispiel 3 und nicht-nachbehandeltes Leuchtstoffes gemäß Beispiel 1, die vorher in Wasser suspendiert waren. Es ist ersichtlich, dass beide Leuchtstoffe nicht hydrolysestabil sind. Das Material Y₂O₃ ist offenbar nicht geeignet, um eine Diffusionsbarriere aufzubauen.

Fig. 3.2 zeigt ein Emissionsspektrum des Beispiels 3 bevor der Leuchtstoff in Wasser suspendiert wurde. Der Leuchtstoff zeigte den gewünschten Wellenlängenbereich.

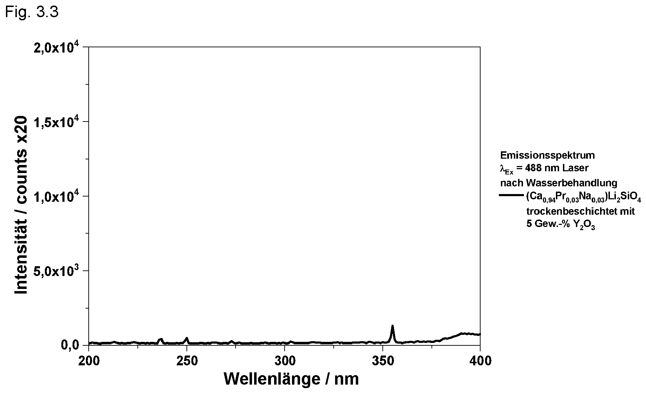

Fig. 3.3 zeigt ein Emissionsspektrum des Beispiels 3 nachdem der Leuchtstoff in Wasser suspendiert wurde. Der Leuchtstoff verlor gänzlich seine Intensität.

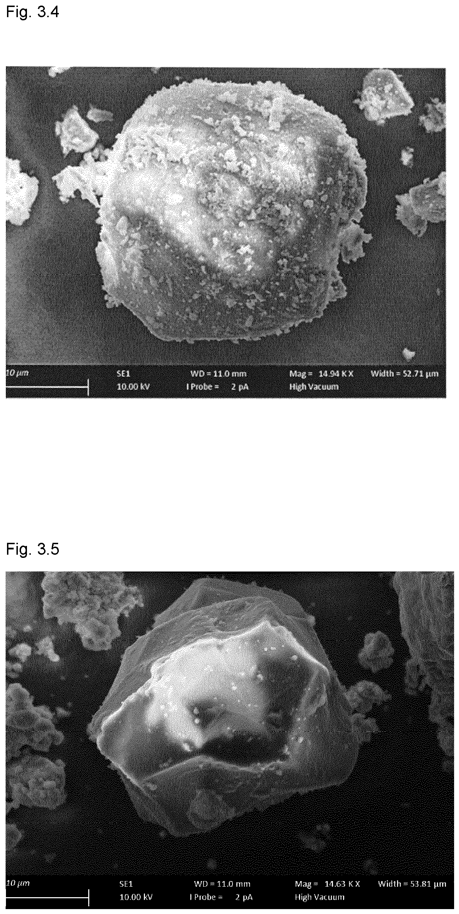

Fig. 3.4 zeigt eine SEM-Aufnahme des Beispiels 3. Die Partikeloberfläche weisen kleine unregelmäßige Flocken auf. Es wird vermutet, dass es sich um aufliegende Y₂O₃ Partikeln handelt.

Fig. 3.5 zeigt eine SEM-Aufnahme von Beispiel 1.1. Die Oberfläche des Leuchtstoffs ist glatter als die des Beispiels 3.

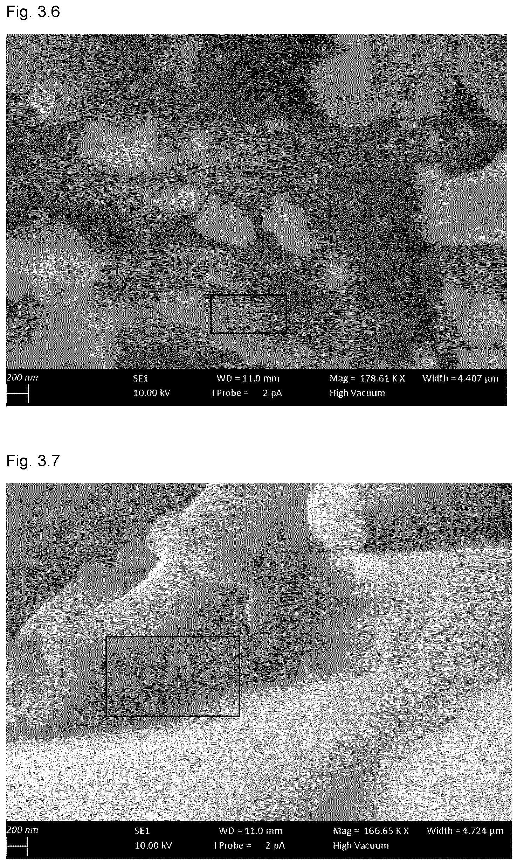

Fig. 3.6 zeigt eine SEM-Aufnahmeeines unbeschichteten Leuchtstoffes, wobei nur ein Ausschnitt (schwarzer Rahmen) betrachtet wird. Es ist zu erkennen, dass neben flockenartigen Bruchstücken des Leuchtstoffs keine Beschichtung auf der Partikeloberfläche zu erkennen ist (siehe schwarzer Rahmen).

Fig. 3.7 zeigt SEM-Aufnahme des Beispiel 1.1. Hier wird ebenfalls nur ein Ausschnitt betrachtet (schwarzer Rahmen). Auf der Oberfläche sind deutliche Erhebungen zu erkennen, die auf die glasartige SiO₂ Beschichtung zurückzuführen sind (siehe schwarzer Rahmen).

### Anwendungsbeispiele

Es wurden analog die Beispiele aus der EP 21157055.1 durchgeführt. Methoden, Geräte und Materialien waren identisch mit denen aus der EP 21157055.1. Es wurde lediglich der erfindungsgemäße Leuchtstoff (nachbehandelt) ersetzt.

### Überprüfung der antimikrobiellen Wirksamkeit

Es wurden wässrige härtbare Zusammensetzungen gemäß Tabelle 1 und Tabelle 2 hergestellt. 50 g Glasperlen wurden zu der jeweiligen Zusammensetzung gegeben und für 5 min bei 2000 rpm im Speedmixer angerieben. Nach Abfiltrieren der Glasperlen wurde die jeweilige Zusammensetzung auf ein hochglanz-gewälztes Alupanel aufgezogen und zu einem Film vernetzt, der eine Trockenschichtdicke von 50 µm aufwies. Auf dem Substrat ist nun eine Beschichtung, deren Beschichtungsoberfläche eine antimikrobielle Wirkung aufweisen soll, sowie deren Referenz ohne erwartete antimikrobielle Wirkung. Die Vergleichsbeispiele VZ1 und VZ2 enthalten keine Leuchtstoffe.

**Tabelle 1**

| | **VZ1 [g]** | **Z1-1 [g]** | **Z21-2 [g]** |
|---|---|---|---|
| Silikopur 8081 (Fa. Evonik) | 91,99 | 91,09 | 90,66 |
| Wasser | 3 | 3 | 3 |
| CaLi₂SiO₄:Pr³⁺,Na⁺(1%) CaF₂, nachbehandelt TEOS/TMOS | | 0,90 | 1,33 |
| TIB Kat^{®} 218 | 0,01 | 0,01 | 0,01 |
| Desmodur^{®} N 3100 | 5 | 5 | 5 |

Silikipur 8081 ist eine lufttrocknende wässrige silikonmodifizierte Polyurethandispersion der Fa. Evonik.

**Tabelle 2**

| | **VZ2 [g]** | **Z2-1 [g]** | **Z2-2 [g]** |
|---|---|---|---|
| Mobilit Idm7991 (wässrige Acrylatdispersion), Fa. Celanese | 82 | 81,1 | 80,67 |
| Dowanol dpnb (Fa. Dow)/Butylglykol-Gemisch (1:1) | 8 | 8 | 8 |
| CaLi₂SiO₄:Pr³⁺,Na⁺(1%) CaF₂, nachbehandelt TEOS/TMOS | | 0,90 | 1,33 |
| Desmodur^{®} N 3100 | 10 | 10 | 10 |

Die Transfermethode wurde analog zu der EP 21157055.1 durchgeführt.

Es wurde festgestellt, dass die erfindungsgemäßen Beschichtungen Z1-1, Z1-2 und Z2-1, Z2-2 antimikrobiell wirken.

## Patentansprüche

1. Wasserbasierte härtbare Zusammensetzung zur Herstellung von Beschichtungen mit anti-mikrobieller Eigenschaft enthaltend
- mindestens ein filmbildendes Polymer,
- optional mindestens ein Additiv,
- optional mindestens einen Härter,
- mindestens einen up-conversion Leuchtstoff der allgemeinen Formel (I)
A_{1-x-y-z}B*_{y}B₂SiO₄:Ln¹ₓ,Ln²_{z}, I
mit
x = 0,0001 - 0,0500;
z = 0,0000 oder z = 0,0001 bis 0,3000 mit der Maßgabe, dass gilt: y = x + z;
A ausgewählt aus der Gruppe bestehend aus Mg, Ca, Sr und Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
Ln¹ ausgewählt aus der Gruppe bestehend aus Praseodym (Pr), Erbium (Er) und Neodym (Nd);
Ln² ausgewählt aus Gadolinium (Gd),
wobei der Leuchtstoff durch eine Nachbehandlung mindestens ein Material, das eine Bandlücke von größer als 6,0 Elektronenvolt (eV) hat und hydrolysestabil ist, aufweist.

2. Zusammensetzung nach Anspruch 1, **dadurch gekennzeichnet, dass** das Material eine Bandlücke von maximal 12 Elektronvolt (eV) aufweist.

3. Zusammensetzung nach einem der vorgenannten Ansprüche, **dadurch gekennzeichnet, dass** das Material ausgewählt ist aus der Gruppe bestehend aus Oxiden, Silikaten, Boraten, Phosphaten eines anorganischen Materials oder einer Mischung davon.

4. Zusammensetzung nach einem der vorgenannten Ansprüche, **dadurch gekennzeichnet, dass** das Material ausgewählt ist aus der Gruppe bestehend aus SiO₂, α-Al₂O₃, MgO, MgAl₂O₄, Ca-polyphosphate, Sr-polyphosphate, Ca- oder Sr-Pyrophosphat (Ca₁₋ₓSrₓ)₃P₂O₇ (mit x = 0,0 bis 1,0) oder einer Mischung davon.

5. Zusammensetzung nach einem der vorgenannten Ansprüche, **dadurch gekennzeichnet, dass** das Material durch eine Nachbehandlung mit einem Ausgangsstoff auf dem Leuchtstoff ausgebildet wurde.

6. Zusammensetzung nach einem der vorgenannten Ansprüche, **dadurch gekennzeichnet, dass** als Ausgangsstoffe ausgewählt ist aus der Gruppe der Tetraalkylorthosilikate, wobei die Alkylgruppen gleich oder verschieden bei jedem Auftreten 1 bis 10 C-Atome, bevorzugt gleich oder verschieden bei jedem Auftreten 1 bis 4 C-Atome aufweisen.

7. Zusammensetzung nach einem der vorgenannten Ansprüche, **dadurch gekennzeichnet, dass** als Ausgangsstoffe ausgewählt ist aus Tetramethyl-, Tetraethyl-, Tetra-n-propyl-, Tetra-iso-propyl- und Tetrabutylorthosilikat und/oder Mischungen davon.

8. Zusammensetzung nach einem der vorgenannten Ansprüche, **dadurch gekennzeichnet, dass** der Leuchtstoff nach der Nachbehandlung einen kristallinen Kern mit einer glasartigen oder amorphen Umhüllung aufweist.

9. Zusammensetzung nach einem der vorgenannten Ansprüche, **dadurch gekennzeichnet, dass** der Leuchtstoff vor der Nachbehandlung mit mindestens einem Flussmittel hergestellt wurde.

10. Zusammensetzung nach einem der vorgenannten Ansprüche, **dadurch gekennzeichnet, dass** als Flussmittel mindestens ein Stoff aus der Gruppe der Ammoniumhalogenide, Alkalimetallhalogenide, Erdalkalimetallhalogenide und Lanthanoidhalogenide eingesetzt wird.

11. Zusammensetzung nach einem der vorgenannten Ansprüche, **dadurch gekennzeichnet, dass** es sich bei den Halogeniden um Fluoride, Bromide oder Chloride handelt.

12. Zusammensetzung nach einem der vorgenannten Ansprüche, **dadurch gekennzeichnet, dass** es sich bei den Alkalimetallen um Kalium, Natrium oder Lithium handelt.

13. Zusammensetzung nach einem der vorgenannten Ansprüche, **dadurch gekennzeichnet, dass** es sich bei den Lanthanoiden um Praseodym handelt

14. Zusammensetzung nach einem der vorgenannten Ansprüche, **dadurch gekennzeichnet, dass** es sich bei den Erdalkalimetallen um Calcium oder Strontium handelt.

15. Zusammensetzung nach einem der vorgenannten Ansprüche, **dadurch gekennzeichnet, dass** der Leuchtstoff mit Praseodym dotiert ist.

16. Zusammensetzung nach einem der vorgenannten Ansprüche, **dadurch gekennzeichnet, dass** der Leuchtstoff mit Praseodym dotiert und mit Gadolinium co-dotiert ist.

17. Zusammensetzung nach einem der vorgenannten Ansprüche, **dadurch gekennzeichnet, dass** der Leuchtstoff eine erstarrte Schmelze aus kristallinen Silikaten oder aus kristallinen Silikaten dotiert mit Lanthanoid-Ionen, umfassend mindestens einen Alkalimetall Ion und mindestens einen Erdalkalimetall Ion, bevorzugt dass die kristalline Silikate mit Praseodym dotiert ist und optional co-dotiert mit Gadolinium, ist.

18. Zusammensetzung nach einem der vorgenannten Ansprüche, **dadurch gekennzeichnet, dass** der Leuchtstoff zumindest partiell kristallin ist.

19. Zusammensetzung nach einem der vorgenannten Ansprüche, **dadurch gekennzeichnet, dass** der Leuchtstoff ausgewählt ist aus Verbindungen der allgemeinen Formel (la)
A_{1-x-y-z}B*_{y}B₂SiO₄:Pr_{x,}Gd_{z}, Ia
mit A ausgewählt aus der Gruppe bestehend aus Mg, Ca, Sr, Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
x = 0,0001 - 0,0500;
z = 0,0000 oder z = 0,0001 bis 0,3000 mit der Maßgabe, dass gilt: y = x + z.

20. Zusammensetzung nach einem der vorgenannten Ansprüche, **dadurch gekennzeichnet, dass** der Leuchtstoff ausgewählt ist aus Verbindungen der allgemeinen Formel (II)
(Ca₁₋ₐSrₐ)_{1-2b}Ln_{b}Na_{b}Li₂SiO₄ (II)
wobei gilt:
Ln ist ausgewählt aus der Gruppe bestehend aus Praseodym, Gadolinium, Erbium, Neodym, vorzugsweise Praseodym;
a = 0,0000 bis 1,0000, vorzugsweise 0,0000 bis 0,1000, insbesondere 0,0000;
b = 0,0001 bis 1,0000, vorzugsweise 0,0001 bis 0,1000, insbesondere 0,0050 bis 0,0150.

21. Zusammensetzung nach einem der vorgenannten Ansprüche, **dadurch gekennzeichnet, dass** der Leuchtstoff ausgewählt ist aus Verbindungen der allgemeinen Formel (IIa)
Ca_{1-2b}Pr_{b}Na_{b}Li₂SiO₄ (IIa)
mit b = 0,0001 bis 1, vorzugsweise 0,0001 bis 0,1, insbesondere 0,005 bis 0,015.

22. Zusammensetzung nach einem der vorgenannten Ansprüche, **dadurch gekennzeichnet, dass** der Leuchtstoff Ca_{0,98}Pr_{0,01} Na_{0,01} Li₂SiO₄ oder Ca_{0,94}Pr_{0,03}Na_{0,03}Li₂SiO₄ ist.

23. Zusammensetzung nach einem der vorgenannten Ansprüche, **dadurch gekennzeichnet, dass** der Leuchtstoff ein Halogen, entsprechend dem Halogenid des Flussmittels, aufweist.

24. Zusammensetzung nach einem der vorgenannten Ansprüche, **dadurch gekennzeichnet, dass** der Leuchtstoff, der bei Bestrahlung mit elektromagnetischer Strahlung mit geringerer Energie und höherer Wellenlänge im Bereich von 2000 bis 400 nm, insbesondere im Bereich von 800 bis 400 nm, elektromagnetische Strahlung mit höherer Energie und kürzerer Wellenlänge im Bereich von 400bis 100 nm, bevorzugt im Bereich von 300 bis 200 nm emittiert, wobei die Intensität des Emissionsmaximums der elektromagnetischen Strahlung mit höherer Energie und kürzerer Wellenlänge eine Intensität von mindestens 1 • 10³ counts/(mm²*s), bevorzugt höher als 1 • 10⁴ counts/(mm²*s), besonders bevorzugt höher als 1 • 10⁵ counts/(mm²*s).

25. Zusammensetzung nach einem der vorgenannten Ansprüche, **dadurch gekennzeichnet, dass** der Leuchtstoff gemäß Formel (II) XRPD Reflexe im Bereich von 23° 2Θ bis 27° 2Θ und von 34° 2Θ bis 39.5° 2Θ aufweist.

26. Zusammensetzung nach einem der vorgenannten Ansprüche, **dadurch gekennzeichnet, dass** das filmbildende Polymer funktionelle Gruppen, vorzugsweise acide Wasserstoffe, enthält, welche mit einem isocyanathaltigen Härter oder mit einem Katalysator reaktiv sind.

27. Zusammensetzung nach einem der vorgenannten Ansprüche, **dadurch gekennzeichnet, dass** das filmbildende Polymer aus der Gruppe der hydroxyfunktionellen Acrylatpolymere, hydroxyfunktionellen Polyesterpolymere, und/oder hydroxyfunktionellen Polyetherpolymere, hydroxyfunktionellen Cellulosederivate, aminofunktionellen Asparticpolymer oder Polyesterpolymere ausgewählt ist, das mit einem isocyanathaltigen Härter reagiert.

28. Zusammensetzung nach einem der vorstehenden Ansprüche, **dadurch gekennzeichnet, dass** das filmbildende Polymer resonanzarm ist.

29. Zusammensetzung nach einem der vorstehenden Ansprüche, **dadurch gekennzeichnet, dass** die Transmission des filmbildenden Polymers bei mindestens 75%, bevorzugt mindestens 80% und besonders bevorzugt mindestens 85%, mittels eines UV/VIS-Zweistrahlspektrometers liegt.

30. Zusammensetzung nach einem der vorstehenden Ansprüche, **dadurch gekennzeichnet, dass** die Transmission mindestens 70%, bevorzugt mindestens 75% und besonders bevorzugt mindestens 80%, mittels eines UV/VIS-Zweistrahlspektrometers beträgt.

31. Zusammensetzung nach einem der vorgenannten Ansprüche, **dadurch gekennzeichnet, dass** der Leuchtstoff eine durchschnittliche Partikelgröße von d50 = 0,1 - 50 µm, bevorzugt d50 = 0,1 - 25 µm, besonders bevorzugt d50 = 0,1 µm - 5 µm, gemessen nach ISO 13320:2020 und USP 429 aufweist.

32. Zusammensetzung nach einem der vorgenannten Ansprüche, **dadurch gekennzeichnet, dass** die Additive ausgewählt sind aus der Gruppe der Dispergiermittel, Rheologiehilfsmittel, Verlaufsmittel, Netzmittel, Entschäumer und UV-Stabilisierungsmittel.

33. Zusammensetzung nach einem der vorgenannten Ansprüche, **dadurch gekennzeichnet, dass** der Härter ausgewählt ist aus der Gruppe der aliphatischen oder cycloaliphatische Isocyanate.

34. Zusammensetzung nach einem der vorgenannten Ansprüche, **dadurch gekennzeichnet, dass** daraus hergestellte Beschichtungen eine antimikrobielle Wirkung gegen Bakterien, Hefen, Schimmelpilze, Algen, Parasiten, Sporen oder Viren aufweisen.

35. Zusammensetzung nach einem der vorgenannten Ansprüche, **dadurch gekennzeichnet, dass** daraus hergestellte Beschichtungen eine antimikrobielle Wirkung gegen
- Erreger nosokomialer Infektionen, vorzugsweise gegen *Enterococcus faecium, Staphylococcus aureus, Klebsiella pneumoniae, Acinetobacter baumannii, Pseudomonas aeruginosa, Escherichia coli, Enterobacter, Corynebacterium diphteria, Candida albicans,* Rotavirus, Bakteriophagen;
- Pathogene Umweltorganismen, vorzugsweise gegen *Cryptosporidium parvum, Giardia lamblia,* Amöben *(Arcanthamoeba* spp., *Naegleria* spp.), *E. coli,* coliforme Bakterien, Fäkalstreptokokken, *Salmonella* spp., *Shigella* spp., *Leginonella* spec., *Pseudomonas aeruginosa, Mykobakteria* spp., enterale Viren (z.B. Polio- und Hepatitis-A Virus);
- Pathogene in Lebensmitteln, vorzugsweise gegen*Bacillus cereus, Campylobacter* spp., *Clostridium botulinum, Clostridium perfringens, Cronobacter* spp., *E. coli, Listeria monocytogenes, Salmonella* spp., *Staphylococcus aureus, Vibrio* spp., *Yersinia enterocolitica,* Bakteriophagen,
aufweisen.

36. Verwendung der Zusammensetzung nach einem der vorgenannten Ansprüche zur Herstellung von Dispersionen, Mahlgütern, Klebstoffen, Spachtelmassen, Putzen, Farben, Lacken oder Drucktinten, Inkjets, Anreibeharzen oder Pigmentkonzentraten.

37. Verwendung der Zusammensetzung nach einem der Ansprüche 1 bis 32 zur Herstellung von Beschichtungen mit anti-mikrobieller Eigenschaft.

38. Verwendung der Zusammensetzung nach einem der Ansprüche 1 bis 32 zur Beschichtung von Substraten in Hygieneeinrichtungen, Krankenhäusern und in der Lebensmittelindustrie.

39. Gegenstand, **dadurch gekennzeichnet, dass** er mindestens teilweise, vorzugsweise vollständig, mit der härtbaren Zusammensetzung nach einem der Ansprüche 1 bis 32 beschichtet ist.

40. Leuchtstoff nach einer Formel (I), (Ia), (II) oder (IIa), **dadurch gekennzeichnet, dass** der der Leuchtstoff einen im wesentlichen kristallinen Kern mit einer glasartigen Umhüllung aufweist.

41. Leuchtstoff nach Anspruch 40, **dadurch gekennzeichnet, dass** die glasartige Umhüllung aus einem Material mit einer Bandlücke von größer als 6.0 Elektronenvolt (eV) besteht.

42. Leuchtstoff nach einem der Ansprüche 41, **dadurch gekennzeichnet, dass** die glasartige Umhüllung aus einem Material mit einer Bandlücke von kleiner als 12.0 Elektronenvolt (eV) besteht.

43. Leuchtstoff nach einem der Ansprüche 40 - 42, **dadurch gekennzeichnet, dass** das Material ausgewählt ist aus der Gruppe bestehend aus Oxiden, Silikaten, Boraten, Phosphaten eines anorganischen Materials oder einer Mischung davon.

44. Leuchtstoff nach einem der Ansprüche 40 - 43, **dadurch gekennzeichnet, dass** das Material ausgewählt ist aus der Gruppe bestehend aus SiO₂, α-Al₂O₃, MgO, MgAl₂O₄, Ca-polyphosphate, Sr-polyphosphate, Ca- oder Sr-Pyrophosphat (Ca₁₋ₓSrₓ)₃P₂O₇ (mit x = 0,0 - 1,0) oder einer Mischung davon.

45. Leuchtstoff nach einem der Ansprüche 40 - 44, **dadurch gekennzeichnet, dass** der kristalline Kern mit mindestens einem Flussmittel ausgewählt aus einem der Ansprüche 6 - 10 hergestellt wurde.
